(19)

**Europäisches Patentamt**
**European Patent Office**
**Office européen des brevets**

(11) **EP 4 230 110 A1**

(12) **EUROPEAN PATENT APPLICATION**
published in accordance with Art. 153(4) EPC

(43) Date of publication:
**23.08.2023  Bulletin 2023/34**

(21) Application number: **21880195.9**

(22) Date of filing: **14.10.2021**

(51) International Patent Classification (IPC):
**A61B 1/018** (2006.01)   **A61B 17/00** (2006.01)
**A61B 17/29** (2006.01)   **A61B 17/295** (2006.01)

(52) Cooperative Patent Classification (CPC):
**A61B 17/320092; A61B 1/018; A61B 17/00;
A61B 17/29; A61B 17/295;** A61B 2017/2825;
A61B 2017/2926

(86) International application number:
**PCT/JP2021/038094**

(87) International publication number:
**WO 2022/080460 (21.04.2022 Gazette 2022/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **14.10.2020  JP 2020173294**

(71) Applicant: **FUJIFILM Corporation
Tokyo 106-8620 (JP)**

(72) Inventors:
• **HIGASHI, Kohei**
  **Ashigarakami-gun, Kanagawa 258-0022 (JP)**
• **NAKAMURA, Koichiro**
  **Ashigarakami-gun, Kanagawa 258-0022 (JP)**

(74) Representative: **Meissner Bolte Partnerschaft
mbB
Widenmayerstrasse 47
80538 München (DE)**

(54) **ULTRASONIC TREATMENT TOOL AND ENDOSCOPE SYSTEM**

(57)    There are provided an ultrasonic treatment tool and an endoscope system with which it is possible to improve an efficiency of transmitting ultrasonic vibration to a structure such as a blood vessel and absorbing the ultrasonic vibration by the structure, and to suppress a temperature rise of a device by increasing an efficiency of conversion into ultrasonic vibration with respect to supplied power.

An ultrasonic treatment tool (18) includes a grip part (33) that grips a structure in a subject; and an ultrasound oscillator unit (34) that is provided in the grip part (33) and disposed at a position facing the gripped structure. A piezoelectric material constituting the ultrasonic vibration unit (34) has a mechanical quality coefficient of 500 or more.

FIG. 3A

FIG. 3B

**Description**

**BACKGROUND OF THE INVENTION**

1. Field of the Invention

The present invention relates to an ultrasonic treatment tool and an endoscope system.

2. Description of the Related Art

In a medical field, it is known that various treatments are performed on a subject by using an ultrasonic treatment tool that generates ultrasonic vibration. WO2019/055870A discloses an ultrasonic treatment tool comprising a pair of grip parts for gripping a structure such as a blood vessel and an ultrasound oscillator (ultrasonic transducer), and configured to transmit ultrasonic vibration through the grip parts and to perform a treatment such as sealing or incision of the blood vessel or the like. The ultrasound oscillator is composed of various devices such as a piezoelectric material, and converts supplied power into ultrasonic vibration.

**SUMMARY OF THE INVENTION**

In the ultrasonic treatment tool as disclosed in WO2019/055870A, there is a need to raise a temperature of the grip part in a short time to about 60°C to 90°C, which is a denaturation temperature of a protein or the like, in order to seal the blood vessel or the like gripped by the grip parts. In order to raise the temperature of the grip part in a short time, each device in the ultrasonic transducer needs to be supplied with as much power as possible.

However, the power supplied to the ultrasound oscillator is not all converted into ultrasonic vibration, but some of the power is converted into heat generation due to an inefficient transmission of vibration and heat generation due to a dielectric loss. In a case in which a proportion of power that is converted to anything other than ultrasonic vibration, particularly to heat generation, in the supplied power is large, a temperature of the device itself increases excessively while the ultrasound oscillator is being driven, which may cause failure or degradation of the ultrasound oscillator, such as loss of piezoelectricity due to depolarization of the piezoelectric material, or deterioration of an acoustic matching layer.

An object of the present invention is to provide an ultrasonic treatment tool and an endoscope system with which it is possible to improve an efficiency of transmitting ultrasonic vibration to a structure such as a blood vessel and absorbing the ultrasonic vibration by the structure, and to suppress a temperature rise of a device by increasing an efficiency of conversion into ultrasonic vibration with respect to supplied power.

An ultrasonic treatment tool according to an aspect of the present invention comprises: a grip part; and an ultrasound oscillator, in which a piezoelectric material constituting the ultrasound oscillator has a mechanical quality coefficient of 500 or more. The grip part is provided at a distal end portion of the ultrasonic treatment tool and grips a structure in a subject. The ultrasound oscillator is provided in the grip part and disposed at a position facing the structure gripped by the grip part.

It is preferable that a direction of ultrasonic vibration by the ultrasound oscillator is parallel to a gripping direction of the grip part.

It is preferable that the ultrasound oscillator is driven by an AC voltage signal having a frequency of which a difference from a resonance frequency corresponding to a thickness dimension of the piezoelectric material is within 20%. It is preferable that an AC voltage signal for driving the ultrasound oscillator is continuously supplied.

It is preferable that the piezoelectric material is lead zirconate titanate.

It is preferable that the grip part grips a blood vessel as the structure. It is preferable that the ultrasonic treatment tool is an endoscope treatment tool that is inserted into the subject through a forceps channel of an endoscope.

It is preferable that the grip part, the ultrasound oscillator, a tubular flexible sheath, an operation wire provided integrally with the grip part and inserted through the flexible sheath, and an operating part provided at a base end portion of the flexible sheath are provided.

It is preferable that the grip part includes a pair of gripping pieces that grips the structure, at least one of the gripping pieces being provided with the ultrasound oscillator.

An endoscope system according to an aspect of the present invention comprises: the ultrasonic treatment tool described above; a power supply device that outputs an AC voltage signal for driving the ultrasound oscillator; and an endoscope having a forceps channel.

According to the present invention, it is possible to improve an efficiency of transmitting ultrasonic vibration to a structure such as a blood vessel and absorbing the ultrasonic vibration by the structure, and to suppress a temperature rise of a device by increasing an efficiency of conversion into ultrasonic vibration with respect to supplied power.

**BRIEF DESCRIPTION OF THE DRAWINGS**

Fig. 1 is an explanatory diagram showing a configuration of an endoscope system.
Fig. 2 is a front view of an endoscope and an ultrasonic treatment tool according to an aspect of the present invention.
Figs. 3A and 3B are cross-sectional views of a main part of the ultrasonic treatment tool, where Fig. 3A is a cross-sectional view of a main part showing a state in which a grip part is closed, and Fig. 3B is a cross-sectional view of a main part showing a state in which the grip part is opened.
Fig. 4 is a perspective view of the ultrasonic treatment tool in the state in which the grip part is closed.
Fig. 5 is a perspective view of the ultrasonic treatment tool in the state in which the grip part is opened.
Fig. 6 is a cross-sectional view taken along the line VI-VI of Fig. 3A.
Fig. 7A is a schematic diagram relating to a dielectric loss of a piezoelectric material, and Fig. 7B is an equivalent circuit diagram in a case in which the dielectric loss occurs.
Fig. 8 is a block diagram showing an outline of an ultrasonic power supply device.
Fig. 9 is an explanatory diagram showing a state in which a blood vessel gripped by the grip part is treated.

**DESCRIPTION OF THE PREFERRED EMBODIMENTS**

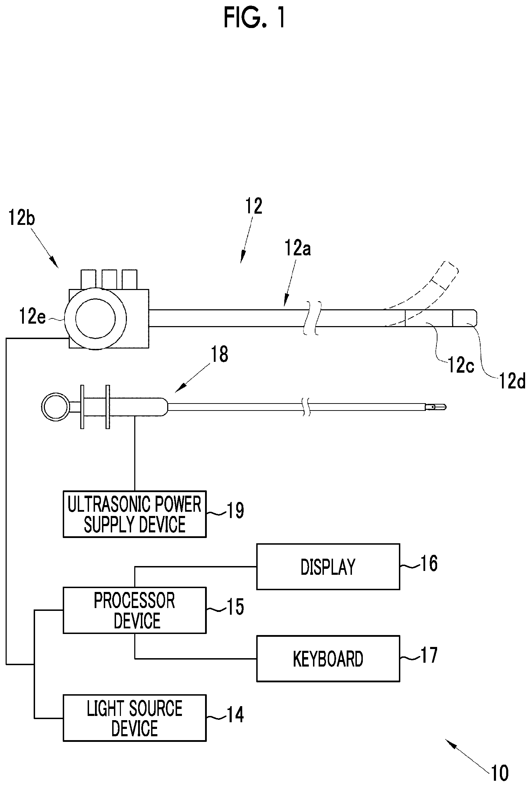

As shown in Fig. 1, an endoscope system 10 comprises an endoscope 12, a light source device 14, a processor device 15, a display 16, a keyboard 17, an ultrasonic treatment tool 18, and an ultrasonic power supply device 19. The endoscope 12 images an observation target. The light source device 14 emits illumination light with which the observation target is irradiated. The processor device 15 performs a system control of the endoscope system 10. The display 16 is a display unit that displays an observation image or the like based on an endoscopic image. The keyboard 17 is an input device that performs setting input to the processor device 15 or the like.

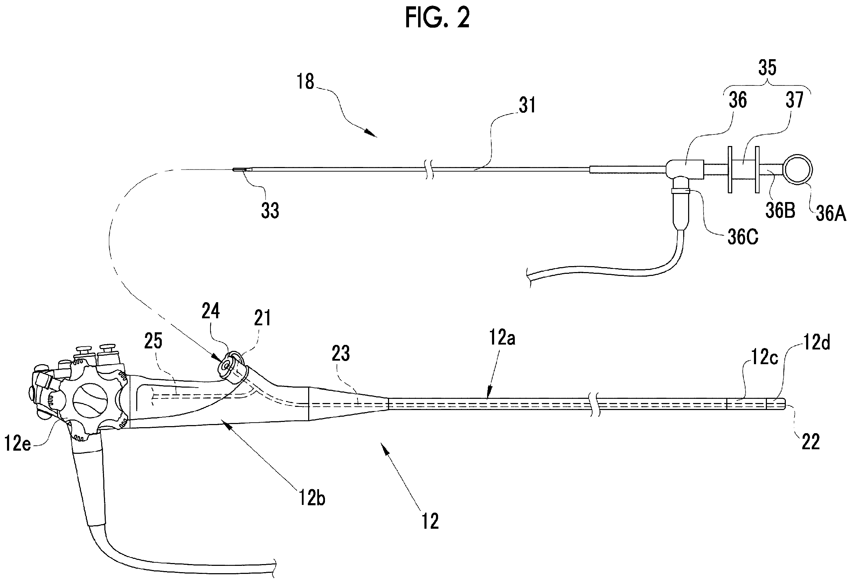

The endoscope 12 is optically connected to the light source device 14 and electrically connected to the processor device 15. The endoscope 12 includes an insertion part 12a to be inserted into a subject, an operating part 12b provided at a base end portion of the insertion part 12a, a bendable part 12c provided on a distal end side of the insertion part 12a, and a distal end portion 12d. By operating an angle knob 12e of the operating part 12b, the bendable part 12c is bent. As a result, the distal end portion 12d is directed in a desired direction. In addition to the angle knob 12e, a forceps port 21 (see Fig. 2) is provided in the operating part 12b. The forceps port 21 is an entrance into which the ultrasonic treatment tool 18 is inserted. The ultrasonic treatment tool 18 inserted into the forceps port 21 protrudes from a forceps outlet 22 (see Fig. 2) of the distal end portion 12d.

An observation window and an illumination window are provided on a distal end surface of the distal end portion 12d, although not shown. An image sensor (not shown) or the like is disposed on the inside of the observation window, and an optical fiber cable (not shown) is disposed on the inside of the illumination window. A signal line of the image sensor and the optical fiber cable are connected to the processor device 15 and the light source device 14, respectively.

The processor device 15 is electrically connected to the display 16 and the keyboard 17. The processor device 15 performs image processing or the like on an endoscopic image captured by the image sensor and displays the processed image on the display 16.

As shown in Fig. 2, a forceps channel 23 for inserting the ultrasonic treatment tool 18 is disposed in the insertion part 12a. One end of the forceps channel 23 is connected to the forceps outlet 22, and the other end of the forceps channel 23 is connected to the forceps port 21 provided in the operating part 12b. The forceps port 21 is provided with a forceps valve 24. The forceps channel 23 is also used as a route for feeding a cleaning liquid such as water from the forceps outlet 22 and a route for sucking a body fluid such as blood and contents such as body waste materials. The ultrasonic power supply device 19 supplies power to an ultrasound oscillator 51, which will be described below, of the ultrasonic treatment tool 18.

The ultrasonic treatment tool 18 is an endoscope treatment tool to be inserted into a subject together with the insertion part 12a through the forceps channel 23. In the present embodiment, the ultrasonic treatment tool 18 is mentioned as the endoscope treatment tool to be combined with the endoscope 12, but, in practice, the present invention is not limited to this, and treatment tools such as biopsy forceps, snares, or electric scalpels are also combined with the endoscope 12.

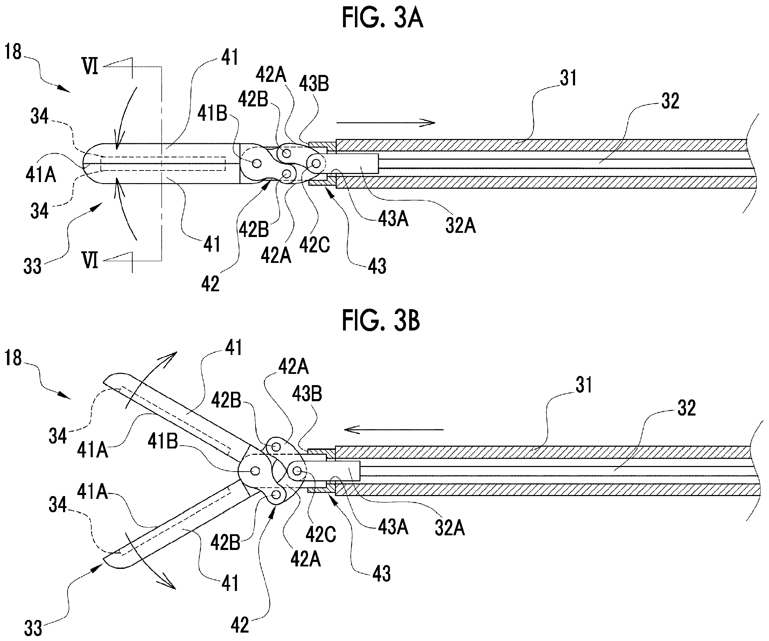

The ultrasonic treatment tool 18 comprises a flexible sheath 31, an operation wire 32 (see Figs. 3A and 3B), a grip part 33, an ultrasound oscillator unit 34 (see Figs. 3A and 3B), and an operating part 35. The flexible sheath 31 is a tubular sheath formed of a flexible material, for example, a soft resin, and is inserted into the forceps channel 23 of the endoscope 12. The operation wire 32 is provided integrally with the grip part 33 and is inserted through the flexible sheath 31.

The operating part 35 comprises an operating part main body 36 and a slider 37 that is slidably supported by the operating part main body 36. The operating part main body 36 is installed consecutively to a base end portion of the flexible sheath 31. The operating part main body 36 is provided with a finger hook portion 36A, a cylindrical portion 36B, and a connector portion 36C. The cylindrical portion 36B extends in a direction parallel to an axial direction of the flexible sheath 31. The slider 37 is engaged with the cylindrical portion 36B and slidably moves along the cylindrical portion 36B in the axial direction of the flexible sheath 31. In a case in which a patient is treated, a thumb of a user is hooked on the finger hook portion 36A, and an index finger and a middle finger of the same user are hooked on the slider 37. A base end of the operation wire 32 is fixed to the slider 37. Therefore, the operation wire 32 is pushed and pulled in the flexible sheath 31 in the axial direction with the sliding movement of the slider 37. The connector portion 36C is electrically connected to the ultrasonic power supply device 19 as described below.

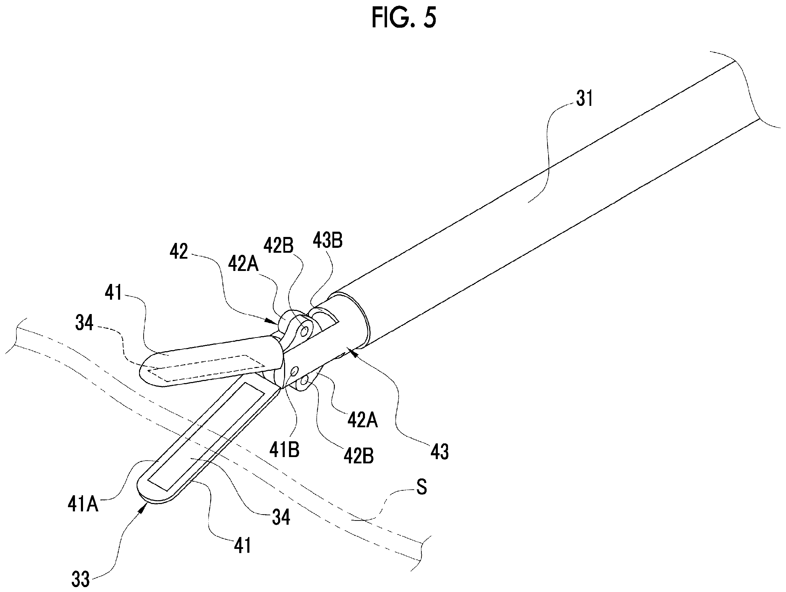

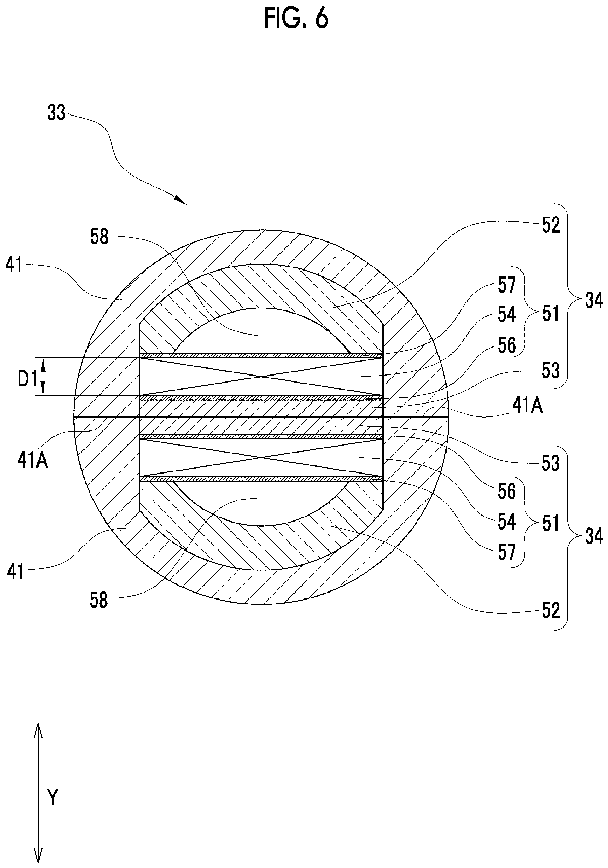

As shown in Figs. 3A and 3B, the grip part 33 is provided at a distal end portion of the ultrasonic treatment tool 18, and comprises a pair of gripping pieces 41 disposed up and down, a link mechanism 42, and a support member 43 that supports the gripping pieces 41 and the link mechanism 42. The ultrasound oscillator unit 34 (see Figs. 5 and 6) is provided on inner surfaces 41A of the pair of gripping pieces 41, that is, surfaces facing each other. In a case in which the grip part 33 is closed, the inner surfaces 41A of the pair of gripping pieces 41 abut on each other. Thereby, a structure such as a blood vessel can be gripped by being interposed between the pair of gripping pieces 41. The pair of gripping pieces 41 constituting the grip part 33 can be opened and closed in a vertical direction about a support shaft 41B. The support shaft 41B is supported by the support member 43. In a case in which the grip part 33 is closed, each of the gripping pieces 41 is formed in a semi-cylindrical shape such that an outer peripheral surface thereof has a continuous cylindrical shape.

The link mechanism 42 comprises a link plate 42A, a connecting pin 42B, and a fitting pin 42C. One end portion of the link plate 42A is connected to the pair of gripping pieces 41 via the connecting pin 42B. A position at which the pair of gripping pieces 41 is connected to the link plate 42A is a base end portion of the gripping pieces 41, which is located on a base end side with respect to the support shaft 41B. The other end portion of the link plate 42A is connected to a connecting member 32A provided at a distal end of the operation wire 32 via the fitting pin 42C. The fitting pin 42C rotatably connects the link plate 42A to the connecting member 32A.

The connecting member 32A is formed in a cylindrical shape. The connecting member 32A is partially located inside the flexible sheath 31 through a through-hole 43A formed in the support member 43. The support member 43 is formed in a substantially cylindrical shape and is fixed to a distal end of the flexible sheath 31. The support member 43 has a notch 43B notched from a distal end thereof. Since the pair of gripping pieces 41 and the link plate 42A move inside the notch 43B, the support member 43 does not hinder the movement of the gripping pieces 41 and the link plate 42A.

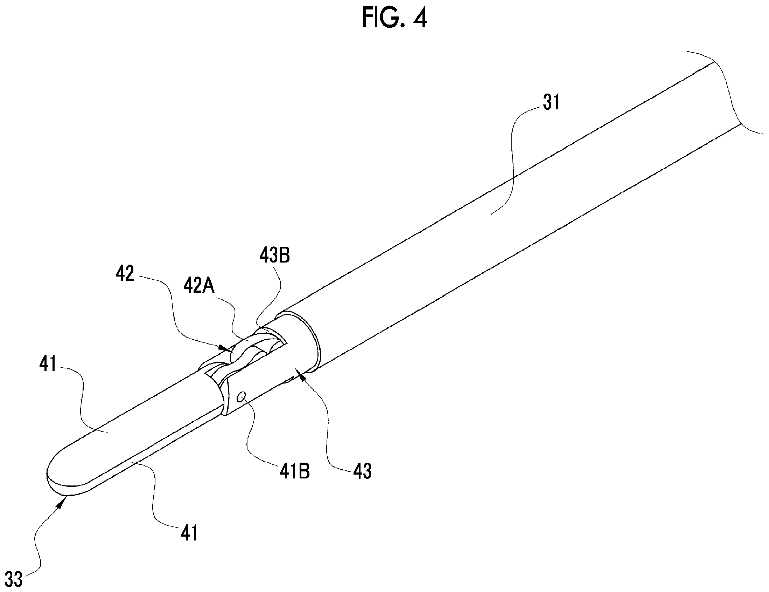

The link mechanism 42 converts a linear motion caused by the pushing-pulling operation of the operation wire 32 into a rotational motion, and opens and closes the gripping pieces 41. That is, in a case in which the slider 37 is pulled toward the finger hook portion 36A, the gripping pieces 41 are closed to close the grip part 33 as shown in Figs. 3A and 4. On the contrary, in a case in which the slider 37 is pushed toward the grip part 33, the gripping pieces 41 are opened to open the grip part 33 as shown in Figs. 3B and 5.

As shown in Fig. 6, the ultrasound oscillator unit 34 is configured by laminating the ultrasound oscillator 51, a backing material layer 52, and an acoustic matching layer 53.

The ultrasound oscillator 51 is composed of a piezoelectric material 54 (also referred to as a piezoelectric element) and electrodes 56 and 57. The piezoelectric material 54 is formed in a plate shape. The electrodes 56 and 57 are formed in a plate shape thinner than the piezoelectric material 54, and are laminated on both surfaces of the piezoelectric material 54. The ultrasound oscillator 51 is disposed in parallel to the inner surface 41A of the gripping piece 41. That is, the ultrasound oscillator 51 is disposed at a position facing a structure such as a blood vessel gripped by the grip part 33. In addition, a direction in which the ultrasound oscillator 51 vibrates is parallel to a direction in which the piezoelectric material 54 and the electrodes 56 and 57 are laminated. Accordingly, a direction of ultrasonic vibration by the ultrasound oscillator 51 is parallel to a gripping direction Y of the grip part 33.

As the piezoelectric material 54, a material having a mechanical quality coefficient Qm of 500 or more is used. The mechanical quality coefficient Qm is a coefficient representing an elasticity loss due to vibration, and is represented by a reciprocal of a mechanical loss coefficient. In a case in which the piezoelectric material 54 elastically vibrates, an internal loss occurs and is converted into heat.

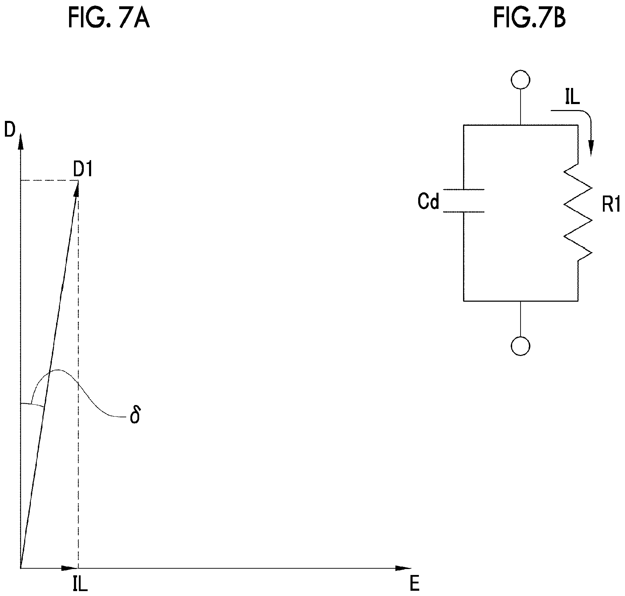

Here, the characteristics of the piezoelectric material 54 will be described. Fig. 7A shows a schematic diagram relating to a dielectric loss of the piezoelectric material 54, and Fig. 7B shows an equivalent circuit diagram of the piezoelectric material 54 in a case in which the dielectric loss occurs. In a case in which a sinusoidal AC electric field E having an angular frequency $\omega$ is applied to the piezoelectric material 54, the piezoelectric material 54 vibrates in a phase in which an electric displacement D1 advances by $\pi/2$ with respect to an electric field E1 in a case of no loss. However, in practice, the electric displacement D1 is delayed by a phase difference $\delta$, and a dielectric loss (tan $\delta$ in [Expression 1]) occurs by the phase difference $\delta$. This dielectric loss causes an action such as dielectric heat generation conversion. The reference numeral IL corresponds to a current supplied to the piezoelectric material 54. There is a relationship of [Expression 1] between an equivalent capacitance Cd, a resonance resistance R1, an angular frequency ω, and a phase difference δ in the equivalent circuit shown in Fig. 7B, and the phase difference δ.

[Expression 1]

$$\tan \delta = \frac{1}{\omega \, C_d R_1}$$

The piezoelectric material 54 has an elasticity loss as well as a dielectric loss. A phase difference of δm is also generated in strain with respect to stress caused by an AC electric field. There is a relationship of [Expression 2] between the phase difference δm due to the elasticity loss and the mechanical quality coefficient Qm.

[Expression 2]

$$\tan \delta_m = \frac{1}{Q_m}$$

Further, the mechanical quality coefficient Qm has a relationship of [Expression 3] between an equivalent capacitance C1, a resonance resistance R1, an angular frequency ωs, an equivalent inductance L1, a resonance impedance Zr, a capacitance C, a resonance frequency fr, and an anti-resonance frequency fa in the equivalent circuit of the piezoelectric material 54 in a case in which elasticity loss occurs.

[Expression 3]

$$Q_m = \frac{1}{\omega_s \, C_1 R_1} = \omega_s \, L_1 \, R_1 \doteqdot \frac{1}{4\pi Z_r \, C \, (f_a - f_r)}$$

In the scope of the present specification and claims, the mechanical quality coefficient Qm defined by [Expression 2] and [Expression 3] is used. The magnitude of the mechanical quality coefficient Qm affects the sharpness of mechanical vibration at the resonance frequency. In the piezoelectric material 54 having a high mechanical quality coefficient Qm, a value of the dielectric loss tan δ that causes heat generation is small, and deterioration of the polarization state is less likely to occur.

That is, in a case in which the mechanical quality coefficient Qm is low, a high vibration velocity cannot be obtained because vibration causes heat generation, but, in a case in which the mechanical quality coefficient Qm is high, a loss is small inside the piezoelectric material 54. That is, it is possible to efficiently convert the supplied power into ultrasonic vibration. Accordingly, in the piezoelectric material 54 having a high mechanical quality coefficient Qm, heat generation is less likely to occur even in a case in which the piezoelectric material 54 vibrates with large power, and a high vibration velocity can be obtained.

As the piezoelectric material 54, lead zirconate titanate (PZT) is preferably used. The piezoelectric material 54 is not limited to this, as long as it is a piezoelectric material having a high mechanical quality coefficient Qm, for example, hard piezoelectric ceramics other than PZT may be used.

The electrodes 56 and 57 are connected to the connector portion 36C of the operating part 35 via a signal cable (not shown). The signal cable is wired along an inner peripheral surface or an outer peripheral surface of the flexible sheath 31, for example. In a case in which the ultrasonic power supply device 19 is connected to the connector portion 36C, the electrodes 56 and 57 are electrically connected to the ultrasonic power supply device 19 via the signal cable and the connector portion 36C. Of the electrodes 56 and 57, one is connected to the ground via the signal cable or the like, and the other is supplied with power of an AC voltage signal, which is described below, from the ultrasonic power supply device 19.

The acoustic matching layer 53 is provided to achieve acoustic impedance matching between a human body of a patient and the ultrasound oscillator 51. The acoustic matching layer 53 is disposed outside the ultrasound oscillator 51, and strictly speaking, as shown in Fig. 6, the acoustic matching layer 53 is superposed on a side facing a structure gripped by the grip part 33 with respect to the ultrasound oscillator 51. That is, the acoustic matching layer 53 is provided at a position exposed from the inner surface 41A of the gripping piece 41.

Since the acoustic matching layer 53 is provided, it is possible to increase a transmittance of the ultrasonic wave. As a material of the acoustic matching layer 53, various organic materials whose acoustic impedance values are closer to that of the human body of the patient than the piezoelectric material of the ultrasound oscillator 48 can be used. Specific examples of the material of the acoustic matching layer 53 include epoxy resin, silicone rubber, polyimide, and polyethylene. In addition, the acoustic matching layer 53 is formed of a plurality of layers, and the material and the number of formed layers are appropriately selected according to a required acoustic impedance value.

The backing material layer 52 supports the ultrasound oscillator 51 from a back side (a side opposite to the acoustic matching layer 53). A backing material is made of, for example, a rigid material such as hard rubber. In addition, an air gap layer 58, that is, a gap interposed between the backing material layer 52 and the ultrasound oscillator 51 is formed between the backing material layer 52 and the ultrasound oscillator 51. Since the air gap layer 58 can reflect the ultrasonic wave by internal air, it has a function of reflecting the ultrasonic wave emitted from the back side of the ultrasound oscillator 51. Accordingly, the ultrasonic vibration can be efficiently transmitted to a structure such as a blood vessel S. Note that the present invention is not limited to this, and the backing material layer 52 may be filled with a material for reflecting the ultrasonic wave without providing the air gap layer 58.

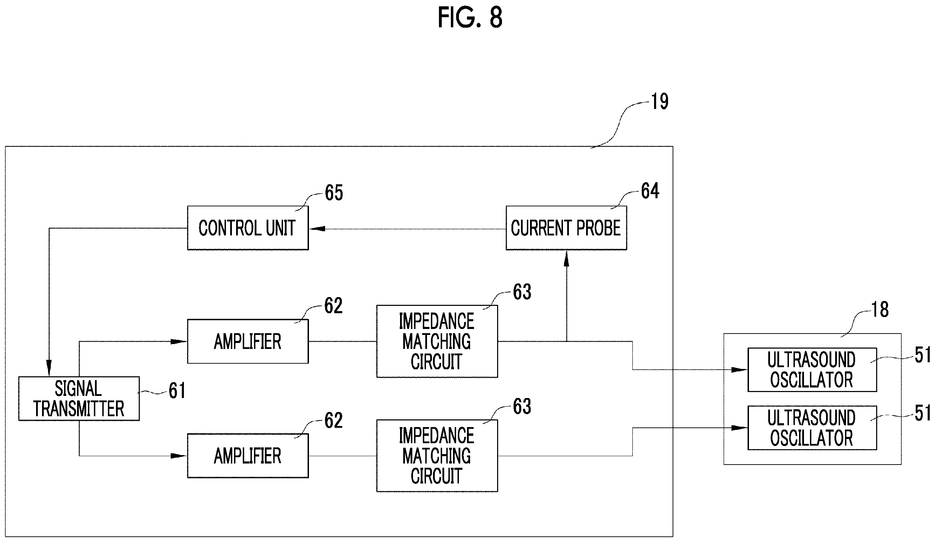

As shown in Fig. 8, the ultrasonic power supply device 19 comprises a signal transmitter 61, an amplifier 62, an impedance matching circuit 63, a current probe 64, and a control unit 65. The signal transmitter 61 has a function of generating an AC voltage signal having an optional frequency and waveform, and has, for example, the same configuration and function as a known function generator.

The signal transmitter 61 outputs an AC voltage signal having a frequency in a predetermined range and a waveform of a sin wave. The frequency in a predetermined range is a frequency of which a difference from a resonance frequency of the piezoelectric material 54 is within 20%. The piezoelectric material 54 has a resonance frequency corresponding to a thickness dimension, and specifically, assuming that the thickness dimension is D1 (m), a resonance frequency corresponding to the thickness dimension D1 is f (Hz), and a sound velocity of the piezoelectric material 54 (a velocity of a sound wave transmitted through the piezoelectric material 54) is v (m/sec), a relationship of $D1 = v/2f$ is obtained.

In order to supply power to a pair of the ultrasound oscillators 51, the amplifier 62 and the impedance matching circuit 63 are provided for each ultrasound oscillator 51. The signal transmitter 61 outputs AC voltage signals having the same frequency and the same waveform to the amplifiers 62, respectively. The amplifier 62 amplifies the AC voltage signal output from the signal transmitter 61 to a voltage at a level at which the ultrasound oscillator 51 can be driven. The impedance matching circuit 63 is connected in series with the amplifier 62, and can match an input impedance of the AC voltage signal output from the amplifier 62 with an impedance of the ultrasound oscillator 51.

The current probe 64 measures a current value to be input to the ultrasound oscillator 51 from the impedance matching circuit 63, and inputs the current value to the control unit 65. The control unit 65 controls the signal transmitter 61 such that the ultrasound oscillator 51 is driven at a frequency at which the current value measured by the current probe 64 peaks.

In the present embodiment, the control unit 65 first controls the signal transmitter 61 to scan and output the AC voltage signal within a frequency range in which a difference from the resonance frequency corresponding to the thickness dimension D1 of the piezoelectric material 54 described above is within 20%. Within the frequency range in which the scan-output is performed, the frequency at which the current value measured by the current probe 64 peaks is detected, and thereafter, the ultrasound oscillator 51 is driven at the peak frequency.

In addition, the control unit 65 controls the signal transmitter 61 to continuously supply the AC voltage signal for driving the ultrasound oscillator 51. The term "continuously supply" as used herein means that the AC voltage signal is continuously output from the signal transmitter 61 without interruption at least during the driving of the ultrasound oscillator 51.

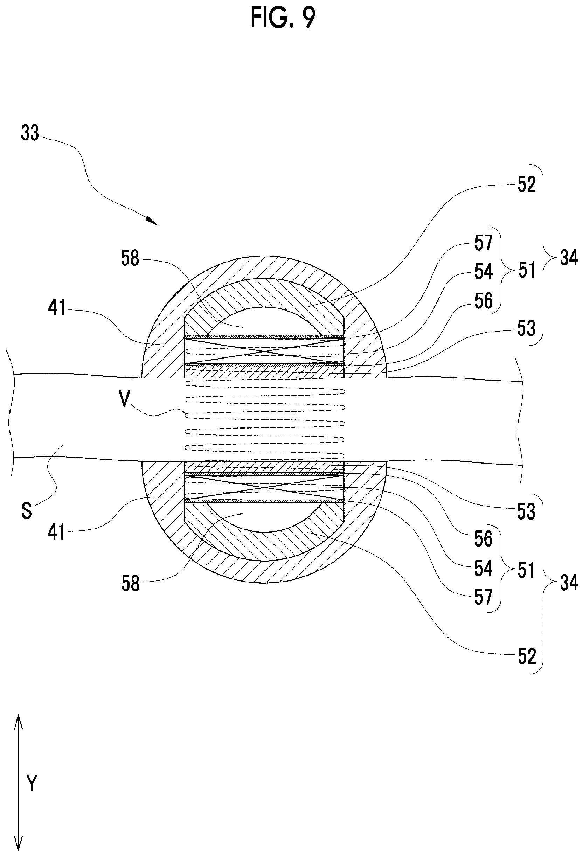

An operation in a case in which a doctor who is a user performs a treatment with the ultrasonic treatment tool 18 using the endoscope system 1 will be described. First, the doctor inserts the insertion part 12a of the endoscope 12 into a body of a patient, which is a subject, and observes an endoscopic image captured by an image sensor to discover, for example, a position of a blood vessel S (see Fig. 5 and Fig. 9), which is a structure in the body of the patient and determine a place to be treated. Then, the doctor inserts the flexible sheath 31 of the ultrasonic treatment tool 18 into the forceps channel 23. In this case, the doctor keeps the grip part 33 closed by operating the operating part 35.

While observing the endoscopic image captured by the image sensor of the endoscope 12, the doctor makes the ultrasonic treatment tool 18 protrude from the forceps outlet 22 and operates the operating part 35 to open the grip part 33. Accordingly, the position of the pair of gripping pieces 41 can be aligned with a position at which the blood vessel S is interposed (state shown in Fig. 5).

Next, the operating part 35 is operated to rotate the gripping pieces 41 from an opened position to a closed position to grip the blood vessel S (state shown in Fig. 9). Further, the doctor operates the ultrasonic power supply device 19 to start the output of the AC voltage signal for driving the ultrasound oscillator 51. Accordingly, the driving of the ultrasound oscillator 51 is started to transmit ultrasonic vibration V to the blood vessel S. In this case, in addition to the ultrasonic vibration V, heat generation such as an electrical loss, which is a small loss but has not been converted into vibration energy, is also transmitted to the blood vessel S gripped by the grip part 33. The ultrasonic vibration V shown by the broken line in Fig. 9 is schematically shown and is different from an actual waveform or the like.

As described above, since the piezoelectric material 54 constituting the ultrasound oscillator 51 has a mechanical quality coefficient Qm of 500 or more, heat generation is less likely to occur in the piezoelectric material 54 even in a case in which the piezoelectric material 54 vibrates with large power, thereby making it possible to obtain a high vibration velocity. That is, it is possible to improve an efficiency of transmitting the ultrasonic vibration to the structure such as the blood vessel S and absorbing the ultrasonic vibration by the structure, and to raise a temperature of the blood vessel S in a short time to about 60°C to 90°C, which is a denaturation temperature of a protein or the like. Accordingly, a treatment such as sealing or incision of the blood vessel S can be easily performed. Further, since the piezoelectric material 54 can efficiently perform conversion into ultrasonic vibration with respect to the supplied power, a temperature rise of the piezoelectric material 54 itself, which is a device, can be suppressed, thereby preventing failure or deterioration of the ultrasound oscillator 51.

In addition, in the present embodiment, since the direction of the ultrasonic vibration V and the gripping direction Y of the grip part 33 are parallel to each other, it is possible to further improve the efficiency of transmitting the ultrasonic vibration V by the ultrasound oscillator 51 to a structure such as a blood vessel and absorbing the ultrasonic vibration V by the structure. In addition, since the ultrasound oscillator 51 is driven by the AC voltage signal having a difference of which a difference from the resonance frequency of the piezoelectric material 54 is within 20%, it is possible to drive the ultrasound oscillator 51 with larger power, thereby obtaining a high vibration velocity.

Hereinafter, verification results for verifying the effects of the present invention will be described. The verification was performed by comparing the piezoelectric material of Examples 1 to 4, which has the mechanical quality coefficient Qm of 500 or more applied to the present invention, with the piezoelectric material of Comparative Examples 1 and 2, which does not have the mechanical quality coefficient Qm of 500 or more applied to the present invention, as the piezoelectric material 54 used for the ultrasound oscillator 51. Table 1 shows the type of the piezoelectric material, the mechanical quality coefficient Qm, and the ultrasonic intensity retention rate in Examples 1 to 4 and Comparative Examples 1 and 2. The piezoelectric materials used in Examples 1 to 4 and Comparative Examples 1 and 2 are piezoelectric ceramics manufactured by Fuji Ceramics Corporation, each of which has a different material. In addition, the ultrasonic intensity retention rate is a ratio of an ultrasonic intensity (proportional to the vibration velocity) in an initial state in which the ultrasound oscillator was driven to an ultrasonic intensity measured after the driving for a certain period of time.

[Table 1]

| | Type of piezoelectric material | Mechanical quality coefficient: Qm | Ultrasonic intensity retention rate (%) |
|---|---|---|---|
| Example 1 | C-213 | 2500 | 90 |
| Example 2 | C-203 | 2000 | 90 |
| Example 3 | C-2 | 1200 | 85 |
| Example 4 | C-204 | 520 | 75 |
| Comparative Example 1 | C-8 | 65 | 40 |
| Comparative Example 2 | C-93 | 67 | 30 |

As shown in Table 1, in Examples 1 to 4 in which the mechanical quality coefficient Qm applied to the present invention is 500 or more, the ultrasonic intensity retention rate is high (ultrasonic intensity retention rate is 75% or more). That is, even in a case in which the ultrasound oscillator composed of the piezoelectric material of Examples 1 to 4 is driven, since the loss is small inside the piezoelectric material, the temperature rise of the device itself is less likely to occur, and the deterioration or the like is less likely to occur, so that a favorable performance can be obtained in that the ultrasound oscillator 51 can be driven with large power.

On the other hand, in Comparative Examples 1 and 2 in which the mechanical quality coefficient Qm applied to the present invention is 500 or more, the ultrasonic intensity retention rate is low (ultrasonic intensity retention rate is 40% or less). That is, in a case in which the ultrasound oscillator composed of the piezoelectric material of Comparative Examples 1 and 2 is driven, since the loss is large inside the piezoelectric material, the temperature of the device itself tends to rise, which may cause deterioration or the like.

From the above description, it has been confirmed that the ultrasonic treatment tool 18 having a favorable performance can be obtained by carrying out the present invention.

In the above-described embodiment, the ultrasound oscillator 51 is provided on both of the pair of gripping pieces 41 constituting the grip part 33, and the ultrasonic vibration is transmitted to the structure from the both ultrasound oscillators 51, but the present invention is not limited to this, and the ultrasound oscillator 51 may be provided only on any one of the pair of gripping pieces 41 constituting the grip part 33. In this case, it is preferable that the ultrasound oscillator 51 is disposed parallel to the inner surface 41A of one gripping piece 41, and that the inner surface 41A of the other gripping piece 41 facing the ultrasound oscillator 51 is formed of a material for reflecting the ultrasonic vibration by the ultrasound oscillator 51.

In addition, in the above-described embodiment, the endoscope 12 to be combined with the ultrasonic treatment tool of the embodiment of the present invention is not specified, and the endoscope 12 need only comprise a forceps channel into which the treatment tool is inserted, for example, a bronchoscope, an upper gastrointestinal endoscope, or a lower gastrointestinal endoscope.

Explanation of References

10: endoscope system
12: endoscope
12a: insertion part
12b: operating part
12c: bendable part
12d: distal end portion
12e: angle knob
14: light source device
15: processor device
16: display
17: keyboard
18: ultrasonic treatment tool
19: ultrasonic power supply device
21: forceps port
22: forceps outlet
23: forceps channel
24: forceps valve
31: flexible sheath
32: operation wire
32A: connecting member
33: grip part
34: ultrasound oscillator unit
35: operating part
36: operating part main body
36A: finger hook portion
36B: cylindrical portion
36C: connector portion
37: slider
41: support piece
41A: inner surface
41B: support shaft
42: link mechanism
42A: link plate
42B: connecting pin
42C: fitting pin
43: support member

43A: through-hole
43B: notch
51: ultrasound oscillator
52: backing material layer
53: acoustic matching layer
54: piezoelectric material
56: electrode
57: electrode
58: air gap layer
61: signal transmitter
62: amplifier
63: impedance matching circuit
64: current probe
65: control unit
D1: thickness dimension
E1: electric field
fa: anti-resonance frequency
f: resonance frequency
fr: resonance frequency
IL: current
L1: equivalent inductance
PZT: lead zirconate titanate
Qm: mechanical quality coefficient
R1: resonance resistance
S: blood vessel
tan $\delta$: dielectric loss
V: ultrasonic vibration
Y: gripping direction
V: ultrasonic vibration
Y: gripping direction
Zr: resonance impedance
$\delta$: phase difference
$\delta$m: phase difference
$\omega$: angular frequency
$\omega$s: angular frequency

**Claims**

1. An ultrasonic treatment tool comprising:

   a grip part that is provided at a distal end portion of the ultrasonic treatment tool and grips a structure in a subject; and
   an ultrasound oscillator that is provided in the grip part and disposed at a position facing the structure gripped by the grip part,
   wherein a piezoelectric material constituting the ultrasound oscillator has a mechanical quality coefficient of 500 or more.

2. The ultrasonic treatment tool according to claim 1,
   wherein a direction of ultrasonic vibration by the ultrasound oscillator is parallel to a gripping direction of the grip part.

3. The ultrasonic treatment tool according to claim 1 or 2,
   wherein the ultrasound oscillator is driven by an AC voltage signal having a frequency of which a difference from a resonance frequency corresponding to a thickness dimension of the piezoelectric material is within 20%.

4. The ultrasonic treatment tool according to any one of claims 1 to 3,
   wherein an AC voltage signal for driving the ultrasound oscillator is continuously supplied.

5. The ultrasonic treatment tool according to any one of claims 1 to 4,
   wherein the piezoelectric material is lead zirconate titanate.

6. The ultrasonic treatment tool according to any one of claims 1 to 5,
   wherein the grip part grips a blood vessel as the structure.

7. The ultrasonic treatment tool according to any one of claims 1 to 6,
   wherein the ultrasonic treatment tool is an endoscope treatment tool that is inserted into the subject through a forceps channel of an endoscope.

8. The ultrasonic treatment tool according to claim 7,
   wherein the grip part, the ultrasound oscillator, a tubular flexible sheath, an operation wire provided integrally with the grip part and inserted through the flexible sheath, and an operating part provided at a base end portion of the flexible sheath are provided.

9. The ultrasonic treatment tool according to any one of claims 1 to 8,
   wherein the grip part includes a pair of gripping pieces that grips the structure, at least one of the gripping pieces being provided with the ultrasound oscillator.

10. An endoscope system comprising:

    the ultrasonic treatment tool according to any one of claims 1 to 9;
    a power supply device that outputs an AC voltage signal for driving the ultrasound oscillator; and
    an endoscope having a forceps channel.

# FIG. 1

ULTRASONIC POWER SUPPLY DEVICE — 19

DISPLAY — 16

PROCESSOR DEVICE — 15

KEYBOARD — 17

LIGHT SOURCE DEVICE — 14

# FIG. 2

EP 4 230 110 A1

FIG. 3A

FIG. 3B

EP 4 230 110 A1

# FIG. 4

EP 4 230 110 A1

# FIG. 5

EP 4 230 110 A1

## FIG. 6

# FIG. 7A

# FIG.7B

EP 4 230 110 A1

## FIG. 8

# FIG. 9

<table>
<tr><td colspan="2" align="center">**INTERNATIONAL SEARCH REPORT**</td><td>International application No.<br>**PCT/JP2021/038094**</td></tr>
</table>

**A.    CLASSIFICATION OF SUBJECT MATTER**

*A61B 1/018*(2006.01)i; *A61B 17/00*(2006.01)i; *A61B 17/29*(2006.01)i; *A61B 17/295*(2006.01)i
FI:    A61B17/00 700; A61B1/018 515; A61B17/29; A61B17/295

According to International Patent Classification (IPC) or to both national classification and IPC

**B.    FIELDS SEARCHED**

Minimum documentation searched (classification system followed by classification symbols)

A61B17/00; A61B17/22; A61B17/225; A61B17/32; A61N7/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y | JP 2015-43879 A (OLYMPUS CORP) 12 March 2015 (2015-03-12)<br>paragraphs [0017]-[0020], [0028]-[0030], [0034]-[0036], [0041]-[0045], fig. 1-6 | 1-4, 6-10 |
| Y | US 2017/0258487 A1 (OLYMPUS CORPORATION) 14 September 2017 (2017-09-14)<br>paragraphs [0040]-[0044], [0052]-[0054], [0059]-[0062], [0071]-[0077], fig. 1-6 | 1-4, 6-10 |
| Y | JP 2011-92727 A (TYCO HEALTHCARE GROUP LP) 12 May 2011 (2011-05-12)<br>paragraphs [0012]-[0015], [0018], [0023], fig. 1-3 | 1-10 |
| Y | 高橋 弘文, チュートリアル－はじめての超音波圧電素子: 圧電材料の選択法, 特性測定法や等価回路, そして応用例について－ はじめての圧電素子の選び方 －圧電セラミックスの特性と選択－, 日本音響学会誌, 2016, vol. 72, no. 5, pp. 244-249, https://www.jstage.jst.go.jp/article/jasj/72/5/72_244/_pdf,<br>columns "2.1 Piezoelectric device", "2.3 PZT piezoelectric ceramics", (TAKAHASHI, Hirofumi. Tutorial: Selecting of the first-time piezoelectric elements: Piezoelectric ceramics characteristic and selecting. Journal of the Acoustical Society of Japan.) | 1-10 |
| Y | US 2014/0005706 A1 (GELFAND, Mark) 02 January 2014 (2014-01-02)<br>paragraph [0203] | 1-10 |

☐ Further documents are listed in the continuation of Box C.          ☑ See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **06 December 2021** | **28 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2021/038094**

| Patent document cited in search report | | | Publication date (day/month/year) | Patent family member(s) | | | Publication date (day/month/year) |
|---|---|---|---|---|---|---|---|
| JP | 2015-43879 | A | 12 March 2015 | (Family: none) | | | |
| US | 2017/0258487 | A1 | 14 September 2017 | (Family: none) | | | |
| JP | 2011-92727 | A | 12 May 2011 | US paragraphs [0021]-[0024], [0027], [0032], fig. 1-3 EP CA | 2011/0098689 2316359 2719180 | A1 A1 A1 | |
| US | 2014/0005706 | A1 | 02 January 2014 | WO CN | 2014/005155 104755010 | A1 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019055870 A